# EUROPÄISCHE PATENTSCHRIFT

(11) **EP 2 010 093 B1**
(45) Veröffentlichungstag und Bekanntmachung des Hinweises auf die Patenterteilung: **11.02.2015**
(21) Anmeldenummer: 07728307.5
(22) Anmeldetag: 19.04.2007
(51) Int. Cl.: A61L 27/50, A61B 6/14, A61C 8/00, A61L 27/18, A61B 6/00, A61B 5/00

(54) **IMPLANTAT, INSBESONDERE KIEFERIMPLANTAT, MIT UNTERSCHIEDLICHEN MATERIALEIGENSCHAFTEN**
IMPLANT, IN PARTICULAR JAW IMPLANT, WITH DIFFERENT MATERIAL PROPERTIES
IMPLANT, EN PARTICULIER IMPLANT DE MÂCHOIRE, À PROPRIÉTÉS DIFFÉRENTES DES MATÉRIAUX

(30) Priorität: 21.04.2006 DE 102006018516
(43) Veröffentlichungstag der Anmeldung: 07.01.2009
(60) Teilanmeldung: 14198900.4
(73) Patentinhaber: Spahn, Frank-Peter, 3500 Hasselt (BE)
(72) Erfinder: Spahn, Frank-Peter, 3500 Hasselt (BE)
(74) Vertreter: Brötz, Helmut
(86) Internationale Anmeldenummer: PCT/EP2007/053848
(87) Internationale Veröffentlichungsnummer: WO 2007/122178

(56) Entgegenhaltungen:
- EP-A1- 0 083 028
- EP-A1- 0 570 172
- CH-A5- 690 416
- DE-A1- 10 334 366
- DE-A1- 19 948 910
- DE-U1- 20 304 367
- DE-U1-202006 003 922
- FR-A- 2 600 523
- US-A- 4 323 080
- US-A- 5 006 984
- US-A- 5 591 233
- US-A1- 2001 038 996
- US-A1- 2005 106 535
- US-A1- 2006 074 311
- US-B1- 6 193 516
- US-B1- 6 641 893
- US-B1- 6 839 457

## Beschreibung

Die Erfindung betrifft ein Verfahren zur Bestimmung von lastabhängigen Verformungen in verschiedenen Knochenbereichen. Die Erfindung betrifft auch ein Implantat, insbesondere ein Kieferimplantat, welches mehrere, jeweils zur Implantation in oder zumindest benachbart zu Knochen geeignete Baubereiche aus nicht oder im Wesentlichen nicht von Knochen resorbierbarem Material aufweist, wobei zwei oder mehr dieser Baubereiche zueinander unterschiedliche Materialeigenschaften, insbesondere zueinander unterschiedliche physikalische Eigenschaften, wie unterschiedliche Verformungs und/oder Festigkeitseigenschaften, aufweisen.

Im Stand der Technik sind Implantate zur Kieferimplantation, d. h. Kieferimplantate , in einteiliger Ausführung aus CH 690416 A5 und in mehrteiliger Ausführung aus DE 19948910 A1 bekannt. Die dort beschriebenen Implantate besitzen ein plattenartiges, zur basalen Verankerung dienendes Fußteil, einen sich aus der Fußteilebene senkrecht erstreckenden Schaft, an dessen freiern, der Fußplatte abgewandten Ende ein sog. Abutment bzw. ein Gewindeabschnitt zur Befestigung eines künstlichen Zahnes oder einer zahnprothetischen Konstruktion vorgesehen ist, sowie ggf. zusätzlich ein oder mehrere ebenfalls plattenartige Etagenteile, die an dem Schaft zwischen der Fußplatte und dem freien Ende gehalten sind. Die Implantate können im Kiefer in dazu formgerecht bspw. gefräste Höhlungen eingesetzt werden und verbleiben dort als möglichst dauerhafte und stabile Verankerung. Um die Stabilität zu begünstigen, können die geometrischen Abmessungen so gewählt werden, dass sich das Implantat durch weite Teile des Kieferquerschnitts erstreckt. In ihren dauerhaft tragenden, d. h. nicht von Knochen resorbierbaren Baubereichen werden diese Implantate aus einem einheitlichen Material, speziell aus Titan, hergestellt, welches eine entsprechende Zertifizierung besitzt. Wenngleich die bekannten Implantate bereits eine Reihe handhabungs- und gebrauchstechnischer Vorteile bieten, kann es unter bestimmten Voraussetzungen nach einer gewissen Gebrauchsdauer des Patienten zu Früh- oder auch noch Spätverlusten des gesamten Implantats kommen, die ihre Ursache in der dynamischen Beanspruchung von Kiefer und Implantat bei Mundöffnung, Mundschluss und Kauakt haben.

Aus Dokument US 6,839,457 B1 ist ein Knochenmessverfahren zur Messung einer Gestalt, Struktur und Architektur auf der Basis von tomographischen Abbildungen unter Anwendung von Röntgenstrahlung und von Belastung bekannt.

US-B1-6 641 893 offenbart einem Kieferimplantat, welches mehrere, jeweils zur Implantation in Knochen geeignete Baubereiche aus nicht oder im Wesentlichen nicht von Knochen resorbierbarem Material aufweist, wobei mehrere dieser Baubereiche zueinander unterschiedliche Elastizität aufweisen, und wobei diese Baubereiche aus unterschiedliche Kompositmaterialen aus Polyetheretherketone und Glas- oder Kohlenstofffasern hergestellt sind.

Davon ausgehend liegt der Erfindung die Aufgabe zugrunde, Implantate der eingangs genannten Art vorteilhaft weiterzubilden, so dass die genannten Nachteile möglichst weitgehend vermieden werden.

Erfindungsgemäß wird vorgeschlagen, dass bei dem Verfahren zum Vergleich Röntgenaufnahmen von Knochenbereichen ohne und mit mechanischer Belastung durchgeführt werden und dass mittels Vergleich der mit und ohne Belastung aufgenommenen Röntgenaufnahmen für einzelne Knochenbereiche jeweils die dort unter der aufgebrachten Belastung stattgefundene Verformung ermittelt wird. Die Röntgenaufnahmen zeigen vorzugsweise jeweils mehrere vergleichsweise interessierende Knochenbereiche gleichzeitig. Zur Erfassung von Verformungen in unterschiedlichen Verformungsrichtungen besteht die Möglichkeit, jeweils vergleichende Röntgenaufnahmen in insbesondere auch unterschiedlichen Aufnahmerichtungen bei entsprechend unterschiedlicher Belastungsrichtung aufzunehmen. Alternativ oder kombinativ können für eine Belastungsrichtung Röntgenaufnahmen auch jeweils ohne und mit mehreren unterschiedlichen Belastungsstärken durchgeführt werden. Das im Rahmen der Erfindung vorgeschlagene Verfahren eignet sich zunächst zur Bestimmung der lokal unterschiedlichen Knochenverformbarkeit, d. h. der örtlichen Verteilung der Verformbarkeit bzw. Nachgiebigkeit. Bevorzugt ist, dass die Röntgenaufnahmen digitalisiert werden (oder sogleich digital erzeugt werden) und rechnergestützt, vorzugsweise unter Verwendung von Bildverarbeitungssoftware, vergleichend zur Bestimmung der lokal unterschiedlichen Verformungen ausgewertet werden. Die Klischees von analogen Aufnahmen können zur Digitalisierung bspw. eingescannt werden. Auf Grundlage der ermittelten, lokal im allgemeinen unterschiedlichen Verformungen kann der Arzt oder bspw. ein Labortechniker das zur Herstellung eines osteomimetischen Implantats für den gewählten Implantationsort am besten geeignete Material auswählen. Insbesondere besteht die Möglichkeit, für verschiedene Baubereiche des Implantats unterschiedliche Materialien oder Materialzusammensetzungen auszuwählen. In diesem Zusammenhang ist bevorzugt, dass im Rahmen des von der Erfindung vorgeschlagenen Verfahrens anhand der ermittelten Verformungen und der zugehörigen Belastungshöhe in den verschiedenen Knochenbereichen jeweils der örtliche Wert einer Materialkenngröße für Verformungs- und/oder Festigkeitseigenschaften, vorzugsweise einer Materialkenngröße zur Charakterisierung des elastischen Knochenverhaltens, weiter vorzugsweise der örtliche Wert des Elastizitätsmoduls, und/oder einer Materialkenngröße für das viskoelastische Knochenverhalten ermittelt wird. Auf diese Weise kann der Praktiker die elastische Qualität des entsprechenden zu implantierenden Knochenbereiches, beispielsweise in einem Ober- oder Unterkiefer feststellen. Zur Anwendung im Rahmen der Herstellung von Kieferimplantaten ist bevorzugt, dass Röntgen-Panorama-Aufnahmen von Kieferbereichen, vorzugsweise von den ein vorhandenes oder geplantes Implantatbett umgebenden Knochenbereichen, mit und ohne Kaubelastung vorgenommen werden. Entsprechende dazu notwendige Messungen können bspw. in radiologischen Praxen vorgenommen werden. Es besteht die Möglichkeit, dass die örtlichen Werte zumindest einer der oben angesprochenen Materialkenngrößen für Verformungs- und/oder Festigkeitseigenschaften, vorzugsweise für den Elastizitätsmodul, für mehrere Randbereiche eines Implantatbettes im Knochen ermittelt werden und diese Werte dann zur Herstellung eines der Knochenumgebung des Implantatbetts osteomimetisch angepassten Implantats, vorzugsweise eines basalen Kieferimplantats, verwendet werden, indem verschiedene, den ausgewerteten Randbereichen des Implantatbettes zugeordnete Baubereiche des Implantats hinsichtlich der Werte der Kenngröße den zugeordneten Randbereichen des Knochens zumindest angenähert oder angepasst werden.

Die Erfindung betrifft gemäß Anspruch 6 ein Implantat der eingangs genannten Art, das zur Lösung der Aufgabe mittels eines Verfahrens gemäß Anspruch 5 hergestellt ist.

Bei dem Implantat ist vorgesehen, dass zwei oder mehr der zur Implantation in oder benachbart zu Knochen geeigneten Baubereiche aus nicht oder im Wesentlichen nicht von Knochen resorbierbarem Material zueinander unterschiedliche Materialeigenschaften, vorzugsweise zueinander unterschiedliche physikalische Eigenschaften, wie unterschiedliche Verformungs- und/oder Festigkeitseigenschaften, aufweisen. Bei den hier angesprochenen Baubereichen eines einoder mehrteiligen Implantats handelt es sich zunächst um solche, die dafür bestimmt bzw. lagemäßig daran angepasst sind, bei der Implantation in Knochenhöhlungen eingesetzt und/oder benachbart oder angrenzend zu Knochen befestigt zu werden. Dies schließt nicht aus, dass erfindungsgemäße Implantate auch weitere, bspw. aus dem Kiefer hervorstehende Baubereiche aufweisen können und dass auch solche Baubereiche mit in die Variation der Materialeigenschaften einbezogen werden. Auch besteht die Möglichkeit, dass zusätzlich auch Baubereiche bspw, an künstlichen Zähnen, zahnprothetischen Konstruktionen usw, die von dem Kieferimplantat getragen werden (d. h. selbst kein Bestandteil des Kieferimplantats sind), mit in die Abstufung der Materialeigenschaften einbezogen werden. Bei den eingangs angesprochenen Baubereichen kann es sich um örtlich unterschiedliche, d. h. voneinander beabstandete und/oder aneinander angrenzende, Implantatbereiche handeln. Die Erfindung geht von der Tatsache aus, dass es sich bei natürlichen Knochen um ein hinsichtlich der physikalischen Eigenschaften, insbesondere der Verformungs- und Festigkeitseigenschaften, inhomogenes Material handelt, d. h. dass in verschiedenen Bereichen des Knochens, insbesondere benachbart oder angrenzend an einen vorgesehenen Implantationsort im Knochen, unterschiedliche Materialeigenschaften vorliegen können. Mit der vorgeschlagenen Lösung schafft die Erfindung die Möglichkeit, die Materialeigenschaften eines Implantates zumindest in bestimmten einzelnen Baubereichen an die lokalen Unterschiede im umgebenden Knochen anzupassen, d. h. ein insofern osteomimetisches Implantat bereit zu stellen. Der Begriff der physikalischen Eigenschaften ist hier in einem weiten Sinn zu verstehen, insofern auch Knochenmaterial in seinem Verhalten insbesondere mit und ohne mechanische Belastung durch verschiedene unterschiedliche Materialeigenschaften zu charakterisieren ist. Im Bereich der Kieferimplantate besteht die Möglichkeit zu dieser Anpassung zwar grundsätzlich auch bei sog. crestalen Implantaten, bevorzugt ist aber an eine Anwendung für sog. basale Kieferimplantate, d. h. für solche mit einem zur Verankerung dienenden Fußteil, gedacht. Über Kieferimplantate hinausgehend ist grundsätzlich eine Anwendung der Erfindung auch auf für andere Körperregionen bestimmte Implantate möglich.

Bevorzugt besteht die Möglichkeit, dass das Implantat in den angesprochenen, zumindest zwei oder mehr verschiedenen Baubereichen zueinander unterschiedliche Elastizität, vorzugsweise einen unterschiedlich hohen Elastizitätsmodul, und/oder unterschiedliches viskoelastisches Verhalten und/oder unterschiedliche Zähigkeit und/oder unterschiedliche Steifigkeit und/oder unterschiedliche Festigkeit, vorzugsweise unterschiedliche Biege- und/oder Torsionsfestigkeit, und/oder unterschiedliche Härte und/oder unterschiedliche Sprödigkeit aufweist. Vor dem Hintergrund, dass in unterschiedlichen Kieferbereichen das elastische Verhalten nicht gleichartig ist, kann erfindungsgemäß bspw. ein Implantat mit in verschiedenen Baubereichen an das elastische Verhalten zugeordneter Knochenbereiche am Implantatbett angepassten elastischen Verhaltens hergestellt werden, d. h. ein Implantat, welches insofern mit dem umgebenden Kieferknochen "isoelastisches Verhalten" zeigt. Eine unterschiedliche Elastizität kann bspw. eine unterschiedliche Höhe des Elastizitätsmoduls, eine in unterschiedlichem Maße linear ausgeprägte Kraft-Verformungs-Beziehung, insbesondere ein gewisses Maß an viskoelastischem Verhalten und insbesondere eine unterschiedlich stark ausgeprägte Fähigkeit zu selbstständigen Rückverformungen nach Deformationen d. h. Federvermögens, einschließen. Als Werkstoff oder Basiswerkstoff zur Herstellung des Implantats zumindest in den besagten Baubereichen ist ein Baumaterial, welches Keton, insbesondere Polyetheretherketon oder eine Mischung aus Polyetheretherketonen, oder einen Kunststoff wie Delrin enthält bevorzugt. In Betracht kommt beispielsweise ein von der Firma Invibio Ltd. unter der Bezeichnung PEEK-OPTIMA^{®} angebotener polyaromatischer, semikristalliner Thermoplast auf Basis von Polyetheretherketonen. Bevorzugt ist das Implantat ganz oder teilweise aus einem Werkstoff hergestellt, der aus Polyetheretherketonen (PEEK) besteht oder diese als Basismaterial mit vorzugsweise überwiegendern Mengenanteil enthält. Neben der für die besagte Anwendung vorteilhaften Biokompatibilität kann dieses Material mittels geeigneter Zusätze den Materialeigenschaften des Knochens, insbesondere dessen Elastizitätsmodul, angepasst werden. Damit können bei dem erfindungsgemäßen Implantat die Überbelastungsreaktionen, die bei herkömmlichen, bspw. aus einem einheitlichen Metallwerkstoff bestehenden Implantaten durch das unterschiedliche Verhalten von Knochen und Implantat unter Belastung erklärbar sind, vermieden werden. Im Rahmen der Erfindung können in verschiedenen Baubereichen des Implantats unterschiedliche Materialeigenschaften bereits durch entsprechende Variationen in der Zusammensetzung des Polyetheretherketons bzw. durch unterschiedliche Kettenlänge oder verschiedene Mischungen mit unterschiedlichen Kettenlängen eingestellt werden. Neben Polyetheretherketonen (Kurzbezeichnung PEEK) sind grundsätzlich auch Polyetherketone und Polyetherketonketone in Betracht zu ziehen, deren jeweilige Strukturformel der Fachliteratur (bspw. Römpp: Chemielexikon) zu entnehmen ist. Zur Verarbeitung solcher Werkstoffe kommen bspw. das Spritzgussverfahren oder andere, dem Fachmann geläufige Verfahren in Betracht. An verschiedenen Baubereichen des Implantats können zur Erzielung unterschiedlicher Materialeigenschaften jeweils unterschiedliches Polyetheretherketon oder jeweils unterschiedliche Mischungen aus Polyetheretherketonen bspw. verschiedener Kettenlängen verwendet werden. Alternativ oder kombinativ kann zur Erzielung unterschiedlicher Materialeigenschaften in verschiedenen Baubereichen des Implantats dort dem Kunststoff, insbesondere dem Polyetheretherketon oder der Mischung aus Polyetheretherketonen, bedarfsweise ein darin verteilter Zusatz beigegeben werden. Als Zusatz bevorzugt sind faserartige Filamente in Form von Kohlenstofffasern und/oder Glasfasern und/oder bspw. Titanfasern, oder andere Stoffe, organische oder anorganische Zusätze mit oder ohne Faserstruktur. Wird bspw, das Material PEEK-OPTIMA^{®} verwendet, könnte für unterschiedliche Kieferbereiche mit abweichendem elastischem Verhalten durch entsprechende Variationen in der Zusammensetzung oder Zusätze ein den jeweiligen Anforderungen gerecht werdendes Material hergestellt werden. Der Vorteil von PEEK besteht für die erfindungsgemäße Anwendung auch darin, dass er sich auch aufgrund seiner biokompatiblen Eigenschaften in Zusammenhang mit Inklusionen unterschiedlicher Art zur Herstellung von Composite-Werkstoffen mit unterschiedlichen Materialeigenschaften eignet. Neben den schon angesprochenen Karbon- oder Glasfasern kommen als Inklusionen auch Titan in Filamentform in Betracht. Mit derartigen Inklusionen lässt sich bspw. die Festigkeit und die Zähigkeit des Composite-Materials beeinflussen, insbesondere erhöhen, und damit in verschiedenen Implantatbaubereichen die physikalischen Eigenschaften an die spezifischen Gegebenheiten im Knochen anpassen. Hinsichtlich der Faserfilamente besteht die Möglichkeit, diese in Form von glatten Fasern und/oder von spiralartig geformten Fasern und/oder von gewindeartig gewendelten Fasern einzusetzen. Während sich durch glatte, d. h. zumindest im Wesentlichen gerade Filamente insbesondere die Stabilität erhöhen lässt, kann durch Zugabe spiralförmiger bzw. gewendelter Filamente das elastische Verhalten verbessert werden, insbesondere die Festigkeit gegen Torsionen verbessert werden. Spiralartige bzw, gewendelte Filamente lassen sich vorzugsweise in Implantatbaubereichen anwenden, in denen wie in dem umgebenden Knochen neben Druck- und Zugkräften gerade auch beim Kauvorgang Scherkräfte auftreten. Nicht homogene Materialien wie z. B. Knochen reagieren auf derartige Krafteinwirkungen mit Verwindung. Sind in die Baubereiche gewendelte bzw. spiralförmige Filamente eingebaut, können diese Baubereiche Verwindungen länger ohne Bruch ertragen als bei Zusatz von glatten Filamenten. Die gewendelten bzw, spiralförmigen Filamente verbessern insofern das elastische Verhalten bzw. die Elastizität des Materials. Gemäß den vorangehenden Ausführungen ist bevorzugt, dass in verschiedenen Baubereichen des Implantats zur Erzielung unterschiedlicher Materialeigenschaften unterschiedliches Polyetheretherketon oder unterschiedliche Mischungen aus Polyetheretherketonen vorgesehen sind und/oder die Art und/oder die Menge an Zusätzen unterschiedlich gewählt ist.

In einer bevorzugten Ausführungsform der Erfindung handelt es sich bei dem Implantat um ein ein- oder mehrteiliges, vorzugsweise um ein basal verankerbares, Kieferimplantat. Denkbar wäre aber auch eine Anwendung auf sog. crestale Implantate ohne basales plattenartiges Fußteil. Hinsichtlich eines basal verankerbaren Kieferimplantats ist bevorzugt, dass dieses zumindest ein basales, vorzugsweise plattenartiges, Fußteil und einen sich von der Fußplatte erstreckenden Schaft aufweist, sowie lediglich vorzugsweise auch ein oder mehrere, an dem Schaft beabstandet von dem Fußteil und vorzugsweise zueinander beabstandet gehaltene Etagenteile, die ebenfalls vorzugsweise plattenartig beschaffen sind. In diesem Zusammenhang werden auch Gestaltungen von Fuß- oder Etagenteilen, die im Wesentlichen aus einem randseitigen Rahmen und in eine mittlere Aussparung ragenden Stegen bestehen, als plattenartig betrachtet. Das Fußteil und ggf. das oder die Etagenteile besitzen eine im Wesentlichen ebene Erstreckung, die vorzugsweise senkrecht zu der des Schafts verläuft. Bevorzugt ist des weiteren, dass das oder die Etagenteile von dem Schaft weniger weit nach außen auskragen als das Fußteil. In Verbindung mit den vorgenannten Merkmalen ist bevorzugt, dass sich die erfindungsgemäßen zwei oder mehr Baubereiche mit zueinander unterschiedlichen Materialeigenschaften an diesen Implantatbestandteilen befinden. Speziell mehrteilige, basal verankerbare Implantate (sog. basale Baukasten-Implantate) eignen sich in besonderer Weise, um dem Implantat in seinen Bauabschnitten unterschiedliche physikalische Eigenschaften zu geben (auch wenn grundsätzlich auch einteilige und crestale Implantate geeignet sind). Betreffend mehrteilige Kieferimplantate lässt sich die Erfindung vorzugsweise auch auf Implantate der aus DE 19948910 A1 bekannten Art zum lateralen Einschub in gefräste Kieferhöhlungen übertragen. Die Offenbarung dieser Druckschrift wird insofern mit in die vorliegende Anmeldung aufgenommen. In diesem Zusammenhang ist bevorzugt, dass der Schaft mit Außengewinde versehen ist, vorzugsweise der Schaft eine oder mehrere Außengewindezonenabschnitte und eine oder mehrere beabstandet benachbarte Glattringzonen aufweist, und dass das Fußteil und/oder das oder die Etagenteile zu dem Außengewinde passendes Innengewinde aufweisen, vorzugsweise Innengewindezonenabschnitte besitzen, die dem Schaft im Bereich seiner Gewindezonenabschnitte in zueinander veränderbarem Abstand zuordbar sind. Auch kann die vorliegende Erfindung unter einer Vielzahl von weiteren Anwendungsmöglichkeiten im Sinne eines bloßen Ausführungsbeispiels auch auf ein einstückiges Implantat angewendet werden, wie es aus CH 690416 A5, deren Offenbarung in diesem Sinne mit in die vorliegende Anmeldung aufgenommen wird, vorbekannt ist. Unabhängig von einer ein- oder mehrteiligen Ausgestaltung des Implantats besteht die Möglichkeit, dass der Schaft und/oder das Fußteil und/oder ein oder mehrere Etagenteile untereinander zumindest bereichsweise unterschiedliche Materialeigenschaften, vorzugsweise zueinander unterschiedliche der vorangehend genannten Eigenschaften, aufweisen. Beispielsweise kann das Fußteil eine höhere Festigkeit als der Schaft und dieser dafür ein ausgeprägteres elastisches Verhalten besitzen. Alternativ oder kombinativ besteht die Möglichkeit, dass der Schaft und/oder das Fußteil und/oder das oder die Etagenteile in sich, d. h. innerhalb ihres jeweiligen Bauabschnitts, Baubereiche mit zueinander unterschiedlichen Materialeigenschaften, vorzugsweise mit zueinander unterschiedlichen der zuvor genannten Materialeigenschaften aufweisen. Beispielsweise kann das Fußteil in einem seiner Randbereiche eine höhere Festigkeit als in seinem z. B. gegenüberliegenden Randbereich besitzen. Bei einer Ausführung, bei welcher der Schaft an einen von dem Fußteilrahmen abstehenden Steg angeschlossen ist, ist zum Beispiel zweckmäßig, wenn der Steg im Vergleich zum übrigen Fußteil vergleichsweise elastischere Materialeigenschaften hat, um nach seitlichen Auslenkungen des Schafts (bspw, durch Kaubewegung) die Rückverformbarkeit zu verbessern. Als zweckmäßig wird auch eine Ausführung erachtet, bei welcher das Kieferimplantat auf einer Seite, die geometrisch zur Implantierung auf der lingualen Kieferseite angepasst ist, steifer ist, insbesondere aus steiferem Material gefertigt ist, als auf der gegenüberliegenden Seite, die insbesondere auch geometrisch vergleichsweise zur Implantierung auf der Kieferwangenseite angepasst ist. In diesem Zusammenhang ist auch bevorzugt, dass der senkrechte Schaft bzw. Gewindeträger wiederum eine geringere Sprödigkeit und höhere Elastizität besitzt als die basale Platte. Damit können die internen Torsionen, Flexionen und Bewegungen bei Mundöffnungen, Mundschluss und Kauakt besser vom Implantatmaterial nachempfunden werden, und das Implantat löst sich folglich nicht so leicht aus dem Implantatbett im Kiefer. Andererseits verletzen sich die durch die internen Bewegungen am Implantatmaterial reibenden Körper/Knochenzellen nicht am zu unelastischen Fremdmaterial. Diese Verletzungen stellten bei Titanimplantaten bisher einen Hauptgrund für Früh- und Spätverluste im gesamten crestalen und basalen Implantatbereich dar. Zur Vermeidung dieser Probleme stellt der die Materialeigenschaften des angrenzenden Knochens, insbesondere die Knochenelastizität, in verschiedenen Baubereichen des Implantats mimetierende Werkstoff eine geeignete Lösung dar. Alternativ oder kombinativ besteht die Möglichkeit, dass in dem basalen plattenartigen Fußteil glatte und/oder spiralförmige und/oder gewindeartig gewendelte Filamente eingeschlossen sind. Glatte Faserfilamente können beispielsweise in Verbindung mit Polyetheretherketon dazu dienen, um eine stabile Basisplatte bei gleichem Elastizitätsmodul wie der umgebende Kieferknochen zu erreichen. Spiralförmige bzw. gewendelte Filamente eigenen sich insbesondere dazu, um die Rückverformung nach Belastung und insofern das elastische Verhalten zu verbessern. Auch eignen sich in der Basisplatte (Fußteil) gewendelte und insbesondere spiralförmige Filamente besser als glatte, um die Festigkeit gegen Torsionen zu verbessern. Auch ist möglich, dass das basale Fußteil einstückig mit einem davon senkrecht ausgehenden Schaft, vorzugsweise mit einem Gewindeträger zur Befestigung eines künstlichen Zahns oder einer prothetischen Überkonstruktion, ausgeführt ist und dass sich in das Fußteil eingeschlossene gewendelte bzw, spiralförmige Filamente bis in den Schaft erstrecken. Solche Filamente können die Auslenkung des Schafts beim Kauakt gewähren und das Zurückfedern in die Ausgangsposition gewährleisten. Auch hier handelt es sich um eine Verbesserung des elastischen Verhaltens und Angleichung an die Elastizität der Körper- bzw. Knochengewebe.

Das von der Erfindung angegebene Verfahren zur Bestimmung von lastabhängigen Verformungen in verschiedenen Knochenbereichen ist insbesondere für die Herstellung der zuvor beschriebenen osteomimetischen Implantate, vorzugsweise Kieferimplantate, nützlich, ist aber auch in anderem Zusammenhang einsetzbar.

Gemäß einer bevorzugten Ausführungsform weist das erfindungsgemäße Implantat als Bestandteile zumindest ein plattenartiges Fußteil und einen Schaft für ein Prothetikteil, ein Abutment oder dergleichen auf, wobei der Schaft an dem Fußteil quer zu einer Haupterstreckungsebene befestigt und/oder befestigbar, insbesondere anschraubbar, ist. Gemäß einer noch weiteren bevorzugten Ausführungsform weist das Implantat als Bestandteile zumindest einen insbesondere stiftartigen Schaft zur Befestigung eines Prothetikteils, eines Abutments oder dergleichen, weiterhin eine erste, vorzugsweise scheibenartige Halterung, zumindest eine zweite, vorzugsweise scheibenartige Halterung und zumindest ein gesondertes, vorzugsweise stiftartiges Verbindungsteil auf, wo bei der Schaft an der ersten Halterung befestigt oder befestigbar, vorzugsweise darin einschraubbar, ist und wobei das Verbindungsteil jeweils an der ersten Halterung und an der zweiten Halterung befestigt oder befestigbar, vorzugsweise darin jeweils einschraubbar ist, zur Verbindung der ersten und der zweiten Halterung in voneinander beabstandeter Anordnung. In diesem Zusammenhang ist bevorzugt, dass die erste und/oder die zweite Halterung jeweils mehrere verteilt daran angeordnete Anschlüsse aufweist, die zum Anschließen des Schafts und/oder eines Verbindungselements geeignet sind. Bevorzugt ist außerdem, dass sich der Schaft im montierten Zustand in seiner Längsrichtung quer, vorzugsweise senkrecht, zu einer Haupterstreckungsebene der ersten Halterung und/oder zu einer Haupterstreckungsrichtung der zweiten Halterung erstreckt. Alternativ oder kombinativ kann sich das Verbindungsteil im montierten Zustand in seiner Längsrichtung, vorzugsweise senkrecht, zu einer Haupterstreckungsebene der ersten Halterung und/oder zu einer Haupterstreckungsrichtung der zweiten Halterung erstrecken. In einer zweckmäßigen Ausgestaltung kann die erste Halterung und/oder die zweite Halterung ein einstückiges Fußteil aufweisen. Zur zweckmäßigen Weiterbildung kann die erste Halterung und/oder die zweite Halterung ein Hülsenteil umfassen, welches eine taschenartige Ausnehmung aufweist, deren Taschenquerschnitt zur Erzielung einer Steckhalterung an einem Fußteil geometrisch an einen Fußteilquerschnitt angepasst ist und wobei das Hülsenteil einen oder mehrere verteilt angeordnete Anschlüsse, die zum Anschließen des Schafts und/oder eines Verbindungsteils geeignet sind, aufweist. Es besteht die Möglichkeit, dass der Schaft und, sofern jeweils vorhanden, ein oder mehrere Verbindungsteile, ein oder mehrere Fußteile und ein oder mehrere Hülsenteile aus einem Werkstoff bestehen, der Kunststoff, vorzugsweise Polyetheretherketon, enthält. Bevorzugt ist an Polyetheretherketon enthaltende Materialien gedacht, deren Elastizitätsmodul zumindest etwa oder näherungsweise in einem für Kieferknochen typischen Wertebereich, beispielsweise im Bereich von 6 bis 12 GPa liegt (also deutlich geringer als der Elastizitätsmodul von Titan ist) und bei deren Verwendung das Implantat die gewünschten, mit dem umgebenden Knochen vergleichbaren physikalischen Eigenschaften erhält. Geeignet sind beispielsweise von der Firma Invibio Ltd. unter den Bezeichnungen PEEK-OPTIMA^{®} und PEEK-CLASSIX^{®} angebotene Werkstoffe. Insofern sind auch Materialien mit einem Elastizitätsmodul von etwa 4 GPa noch gut geeignet. Speziell ist bevorzugt, dass der Werkstoff, aus dem der Schaft und/oder ein oder mehrere Verbindungsteile hergestellt sind, einen niedrigeren, vorzugsweise einen um den Faktor 2 bis 3 niedrigeren, Elastizitätsmodul aufweisen als der Werkstoff, aus dem das oder die Fußteile und, sofern vorhanden, das oder die Hülsenteile, hergestellt sind. Auch in diesem Zusammenhang ist bevorzugt, dass die insofern unterschiedlichen Werte des Elastizitätsmoduls der bei den verschiedenen Implantatkomponenten eingesetzten verschiedenen Materialien jeweils in dem für Knochen typischen Wertebereich des Elastizitätsmoduls, also insbesondere im Bereich von etwa 4 bis 12 GPa liegen. Vorzugsweise weisen das oder die Fußteile und, sofern vorhanden, das oder die Hülsenteile, vergleichsweise weniger elastische Eigenschaft auf als der Schaft und, sofern vorhanden, das oder die Verbindungsteile. Eine zweckmäßige Weiterbildung wird auch darin gesehen, dass der Werkstoff, aus dem das oder die Fußteile und, sofern vorhanden, das oder die Hülsenteile, hergestellt ist, zusätzlich Glasfasern und/oder Kohlenstofffasern und/oder Titanfasern enthält. Geeignet sind beispielsweise die von der Fa. Invibio Ltd. unter den Bezeichnungen PEEK-OPTIMA CA130^{®} und PEEK-OPTIMA CF^{®} angebotenen Materialien. Bei dem Fußteil und den Hülsenteilen kann der Elastizitätsmodul vorzugsweise im Bereich von etwa 11 GPa liegen. Je nach Bedarf bzw. Anforderungen wäre auch die Verwendung von Materialien vorstellbar, deren Elastizitätsmodul im Vergleich zu den zuvor genannten Werten entweder niedriger oder höher ist.

Die Erfindung wird nachfolgend mit Bezug auf die beigefügten Zeichnungen, welche bevorzugte Ausführungebeispiele des Implantats zeigen, weiter beschrieben. Dabei zeigt:
- Fig. 1: perspektivisch ein erfindungsgemäßes, einstückiges Kieferimplantat gemäß einer ersten bevorzugten Ausführungsform;
- Fig. 2: perspektivisch eine weitere bevorzugte Ausführungsform eines Kieferimplantats, jedoch in mehrteiliger Ausgestaltung;
- Fig. 3: das in Fig. 2 gezeigte Kieferimplantat in einem durch dessen Schaft geführten Längsschnitt;
- Fig. 4: perspektivisch eine weitere bevorzugte Ausführungsform eines einstückigen Kieferimplantats;
- Fig. 5: perspektivisch eine weitere bevorzugte Ausführungsform eines mehrteiligen Kieferimplantats;
- Fig. 6: das in Fig. 5 gezeigte Kieferimplantat in einem durch den Schaft geführten Längsschnitt;
- Fig. 7: eine Draufsicht auf das Fußteil des in Fig. 5 dargestellten Implantats;
- Fig. 8: eine Abwandlung des in Fig. 7 dargestellten Fußteils;
- Fig. 9: eine zweite Abwandlung des in Fig. 7 gezeigten Fußteils;
- Fig. 10: perspektivisch ein Fußteil gemäß einer bevorzugten Ausführungsform;
- Fig. 11: das in Fig. 10 gezeigte Fußteil in umgekehrter Lage;
- Fig. 12: perspektivisch ein Fußteil gemäß einer weiteren bevorzugten Ausführungsform;
- Fig. 13: das in Fig. 12 gezeigte Fußteil in umgekehrter Lage;
- Fig. 14: perspektivisch ein erfindungsgemäßes Implantat gemäß einer weiteren bevorzugten Ausführungsform;
- Fig. 15: perspektivisch ein erfindungsgemäßes Implantat gemäß einer weiteren bevorzugten Ausführungsform und
- Fig. 16: das Hülsenteil gemäß Fig. 15.

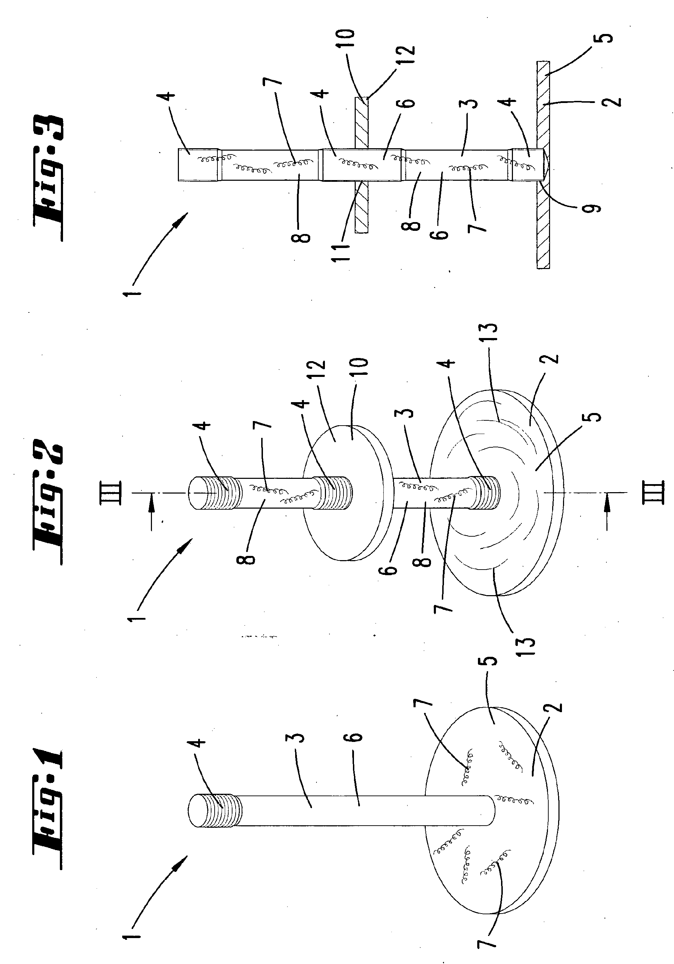

Figur 1 zeigt perspektivisch das erfindungsgemäße Implantat 1 gemäß einer ersten bevorzugten Ausführungsform. Dabei handelt es sich um ein Kieferimplantat mit einem basalen, plattenartigen und in dem Beispiel kreisrunden Fußteil 2 und einem damit einstückig ausgebildeten bzw. verbundenen zylindrischen Schaft 3, der sich von der Fußteilmitte ausgehend in seiner Längsrichtung senkrecht zur Fußteilebene erstreckt. Das Fußteil 2 kann lateral in eine gefräste Kieferhöhlung eingeschoben werden und dient dort zur basalen Verankerung des Kieferimplantats. Der Schaft 3 wird beim Einsetzen des hier beispielhaft gezeigten einstückigen Implantats gleichfalls lateral in eine vorbereitete Kieferhöhlung geschoben, so dass auf dem am oberen Ende vorgesehenen Gewindezonenabschnitt 4 bspw. ein künstlicher Zahn oder eine zahnprothetische Konstruktion befestigt werden kann. Das Implantat 1 ist im Wesentlichen aus einem Baumaterial hergestellt, das überwiegend Polyetheretherketon oder eine Mischung aus sich bspw. in der Kettenlänge oder der Struktur unterscheidenden Polyetheretherketonen enthält und dem ggf. gewisse Zusatzstoffe zur Beeinflussung der Materialeigenschaften, wobei die vorgenannte Zusammensetzung des Basismaterials für das Fußteil 2 und den Schaft 3 hier einheitlich gewählt wurde. In dem in Figur 1 gezeigten Ausführungsbeispiel bildet das Fußteil 2 im Sinne der Erfindung exemplarisch einen ersten Baubereich 5 und der Schaft 3 einen zweiten Baubereich 6 jeweils zur Implantation im Kieferknochen. Damit sich die verschiedenen Baubereiche 5 und 6 in ihren Materialeigenschaften unterscheiden, sind dem Baumaterial im Bereich des Fußteils 2, d. h. des ersten Baubereichs 5, gewindeartig gewendelte faserartige Filamente 7 zugegeben, deren Form, Größe, Menge und Verteilung innerhalb des Fußteils 2 in Figur 1 (wie auch in folgenden Figuren) lediglich schematisch angedeutet ist. Die Filamente 7 können innerhalb des Fußteils 2 entweder gleichmäßig oder ungleichmäßig verteilt angeordnet sein, wobei die Darstellung dies und auch den Mengenanteil nicht qualitativ zum Ausdruck bringt, sondern je nach Anforderungen Abweichungen möglich sind. Durch die in Figur 1 als Inklusionen im Fußteil 2 vorgesehenen gewindeartig gewendelten Filamente 7, bei denen es sich im Beispiel um Kohlenstofffasern handelt, wird ein Composite-Werkstoff gebildet, der andere Materialeigenschaften als das Basismaterial ohne die Filamente 7 besitzt. Speziell wird durch die gewindeartig gewendelten Filamente 7 die Festigkeit gegen Torsionen verbessert, was insbesondere für einen Einsatz des Kieferimplantats im Unterkiefer-Seitenzahnbereich vorteilhaft sein kann.

Figur 2 zeigt perspektivisch eine zweite bevorzugte Ausführungsform des erfindungsgemäßen Implantats 1, wobei (wie auch nachfolgend) für einander vergleichbare Merkmale zur besseren Übersicht die gleichen Bezugszeichen verwendet werden. Ein Unterschied gegenüber Figur 1 besteht darin, dass das in Figur 2 gezeigte Implantat 1 mehrteilig ist. Der Schaft 3 besitzt dazu zusätzlich zu dem am oberen Ende liegenden Gewindezonenabschnitt 4 (bspw. für einen künstlichen Zahn) je einen im Mittenbereich und am unteren Ende befindlichen Gewindezonenabschnitt 4. Die Gewindezonenabschnitte 4 sind durch zwei Glattringzonen 8 voneinander beabstandet, deren Durchmesser etwas geringer als der Gewindekern ist. Zur Befestigung an dem plattenartigen Fußteil 2 ist der Schaft 3 mit dem unteren Gewindezonenabschnitt 4 in einen passenden Innengewindezonenabschnitt 9 (vgl. auch Figur 3) im Fußteil 2 eingeschraubt. Auf den mittleren Gewindezonenabschnitt 4 ist ein im Vergleich zu dem Fußteil 2 weniger weit zu den Rändern hin auskragendes, ebenfalls plattenartiges Etagenteil 10 mittels einem ebenfalls passenden Innengewindezonenabschnitt 11 aufgeschraubt. Das Etagenteil 10 kann dazu bspw. zunächst über den oberen Gewindezonenabschnitt 4 des Schafts 3 hinweg aufgeschraubt, dann über die obere Glattringzone 8 verschoben und anschließend auf den mittleren Gewindezonenabschnitt 4 aufgeschraubt werden, wobei sich insofern eine variable, d. h. einstellbare Beabstandung zu dem Fußteil 2 ergibt. Bei dem in den Figuren 2 und 3 gezeigten zweiten Ausführungsbeispiel kann im Sinne der Erfindung das Fußteil 2 (oder auch bspw. ein Teil desselben) als erster Baubereich 5, der Schaft 3 (oder auch bspw. ein Teil desselben) als zweiter Baubereich 6 und das Etagenteil 10 (oder auch bspw. ein Teil desselben) als dritter Baubereich 12 aufgefasst werden, wobei wiederum einheitlich ein Baumaterial auf Basis von Polyetheretherketonen verwendet ist. Um den verschiedenen Baubereichen unterschiedliche Materialeigenschaften zu geben, sind ihnen unterschiedliche Zusätze beigegeben. Wie schematisch angedeutet, enthält das Baumaterial im Fußteil 2 glatte, d. h. im Wesentlichen gerade verlaufende Filamente 13, durch welche die Stabilität der Basisplatte vergrößert wird und wodurch sich insbesondere eine Anpassung des Elastizitätsmoduls an den umgebenden Kieferknochen erreichen lässt. Im zweiten Baubereich 6 (im Schaft 3) sind dem Baumaterial gewindeartig gewendelte Filamente 7 zugesetzt, um die Möglichkeit von Auslenkungen des Gewindeträgers von bspw. bis zu 150 µm beim Kauakt und des Zurückfederns in die Ausgangsposition zu verbessern und um die notwendige Resistenz gegen Bruch zu erreichen. Insofern lässt sich das elastische Verhalten verbessern und die Elastizität an das umgebende Körpergewebe angleichen. Aufgrund der beschriebenen Merkmale und Eigenschaften bietet das mit Bezug auf die Figuren 2 und 3 beschriebene Implantat 1 unter Berücksichtigung der auftretenden Beanspruchungen insbesondere Vorteile beim Einsatz im Oberkiefer, speziell im Oberkiefer-Seitenzahnbereich. Je nach Anforderungen kann dabei auch auf das Etagenteil 10 verzichtet werden oder ggf. ein weiteres Etagenteil eingesetzt werden. In Figur 3 sind im Fußteil 2 im Hinblick auf die Schraffur die glatten Filamente 13 nicht mit wiedergegeben, die in Figur 2 gewählte schematische Darstellung zeigt aber, dass diese nicht nur an der Oberfläche, sondern auch im Inneren des Fußteils 2 durchgängig vorhanden sind.

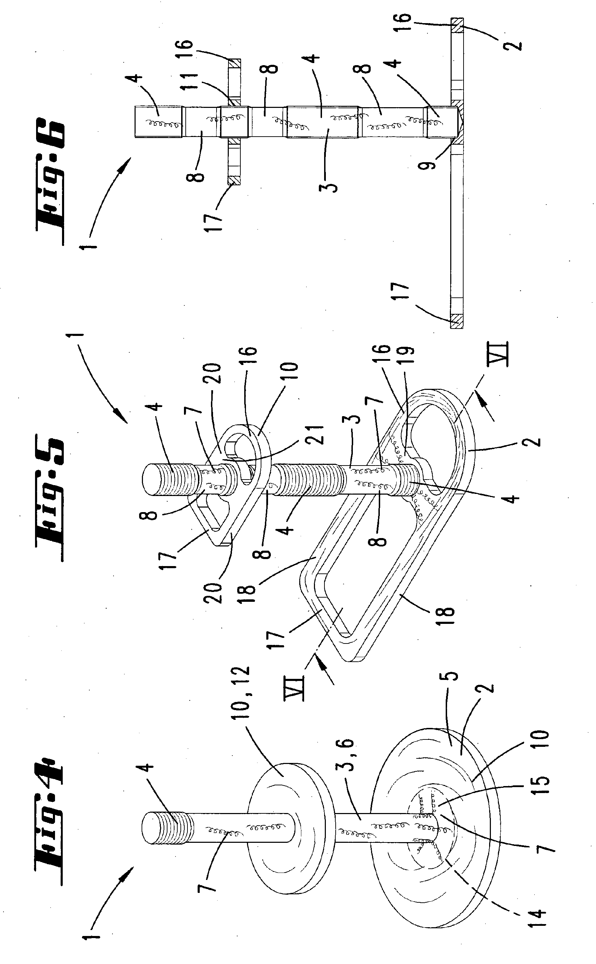

Figur 4 zeigt das erfindungsgemäße Implantat 1 gemäß einer weiteren bevorzugten, wiederum einstückigen Ausführungsform. Die einteilige Herstellung schafft die Möglichkeit, dass sich gewindeartig gewendelte Filamente 7 von dem Fußteil 2 bis in den Schaft 3 hinein durchgehend (ebenfalls einstückig) innerhalb des umgebenden Baumaterials erstrecken. Diese bilden in dem Übergang vom Fußteil 2 zum Schaft 3, der aufgrund des starken Querschnittsüberganges auch bei einteiliger Ausführung relativ bruchempfindlich ist, gleichsam eine elastische Bewehrung und verringern dadurch die Bruchgefahr. Durch die gewendelten Filamente 7 in besagtem Übergangsbereich wird das Auslenkungsvermögen des Schafts 3 beim Kauakt und dessen anschließendes Zurückfedern in die Ausgangsposition unterstützt, d. h. das elastische Verhalten verbessert. Der an das untere Ende des Schafts 3 anschließende, radial innere und von der Bezugskreislinie 14 umschlossene Fußteilbereich, in dem die gewendelten Filamente 7 verankert sind, besitzt im Vergleich zu dem radial außen an die Linie 14 anschließenden Fußteilbereich andere Materialeigenschaften. Insofern können beim Ausführungsbeispiel von Figur 4 die Fußteilbereiche einerseits innerhalb und andererseits außerhalb der Bezugskreislinie 14 als im Sinne der Erfindung verschiedene Baubereiche 5, 15 mit voneinander unterschiedlichen Materialeigenschaften aufgefasst werden. Dies bedeutet, dass sich an dem Fußteil 2 verschiedene Baubereiche mit zueinander unterschiedlichen Materialeigenschaften befinden. Die mit Bezug auf die Figuren 1 bis 4 beschriebenen Implantate sind nicht auf die jeweils dargestellten geometrischen Formen begrenzt, sondern können wie der Schaft auch eine abweichende Formgebung und/oder Größe aufweisen. Insbesondere können die Fuß- und/oder Etagenteile auch in praktisch beliebiger Weise abweichende Konturen und bspw. auch innere und/oder randseitige Ausnehmungen besitzen.

Figur 5 zeigt perspektivisch eine weitere bevorzugte Ausführungsform eines erfindungsgemäßen, mehrteiligen Implantats 1. Hinsichtlich der Mehrteiligkeit und der zur Verbindung der Komponenten gewählten Schraubverbindungen ist die Ausführung mit der in Figur 2 gezeigten Variante zu vergleichen. Unterschiede bestehen jedoch erkennbar in der Ausführung von Fußteil 2 und Etagenteil 10. Auch bei der in Figur 5 gewählten Abwandlung handelt es sich jeweils um einen im Wesentlichen scheibenartigen Flachkörper. Dieser besitzt in beiden Fällen einen etwa hufeisenförmig umlaufenden Randbereich 16, der von einem Außensteg 17 geschlossen wird. Die sich gegenüberliegenden Längsseiten 18 des Fußteils 2 sind durch einen brückenartigen Innensteg 19 und die sich gegenüberliegenden Längsseiten 20 des Etagenteils 10 durch einen brückenartigen Innensteg 20 jeweils paarweise stoffschlüssig verbunden. In der Mitte des Innenstegs 19 befindet sich die im Schnitt von Figur 6 sichtbare Sacklochbohrung mit Innengewindezone 9 zur Schraubverbindung mit dem Schaft 3, während in der Mitte des Innensteges 21 eine Durchgangsbohrung (vgl. Figur 6) mit Innengewindezone 11 eingebracht ist. Zu beiden Seiten des Innenstegs 19 bzw. 21 weist das Fußteil 2 bzw. Etagenteil 10 von dem umlaufenden Rand umfangene Durchgangsöffnungen auf. Der senkrechte Gewindeträger 3, der an dem unteren Gewindezonenabschnitt 4 mit der Basisplatte 2 verbunden ist, ist im Bereich seiner gesamten Länge mit gewindeartig gewendelten Filamenten 7 durchsetzt und kann dadurch mit elastischen Eigenschaften, die der lateralen Auslenkfähigkeit des Zahnhalteapparates nachempfindbar sind, ausgestattet werden. Im Hinblick auf seine Mehrteiligkeit kann das in den Figuren 5 und 6 dargestellte Implantat 1 auch als sog. Baukasten-Implantat bezeichnet werden, wobei bedarfsgerecht auch die Möglichkeit besteht, einzelne oder mehrere Komponenten gegen andere auszutauschen. Alternativ könnte aber auch das in den Figuren 5 und 6 gezeigte Implantat einstückig hergestellt sein.

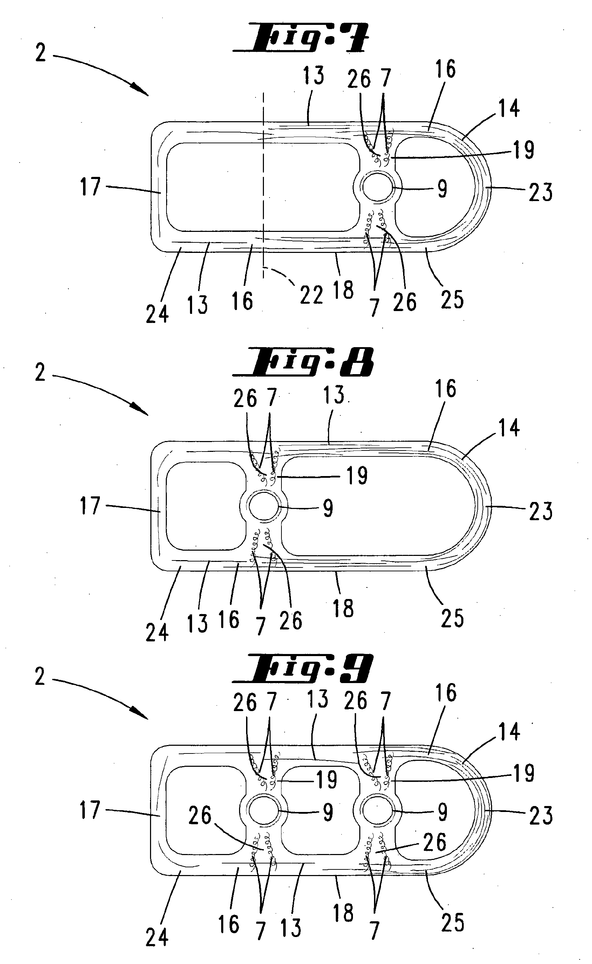

In Figur 7 ist das schon in den Figuren 5 und 6 gezeigte Fußteil 2 in Draufsicht dargestellt. Um dem Fußteil 2 bereichsweise unterschiedliche Materialeigenschaften zu geben, wurden dem als Basismaterial dienenden Polyetheretherketon im hufeisenartig verlaufenden Rand 16 und in dem diesen schließenden Außensteg 17 jeweils glatte Filamente 13 beigegeben. Aus der Darstellung geht schematisch hervor, dass in dem Randbereich, der den Außensteg 17 und den sich von dort bis zu der geometrischen Bezugslinie 22 erstreckenden Teil des hufeisenartigen Randes 16 einschließt, eine geringere Konzentration an geraden Filamenteinschlüssen 13 (Inklusionen) gewählt worden ist als in dem von der Linie 22 bis zum Scheitel 23 der Rundung verlaufenden Randbereich, wobei die Bezugslinie 22 die scheitelabgewandte Durchbrechung des Fußteils 2 etwa hälftig durchquert. Durch die vergleichsweise vermehrte Zugabe von glatten Inklusionen 13 kann der zu dem Scheitel 23 hin abgerundete Teil der Basalplatte bis zu der Bezugslinie 22 weniger elastisch als der jenseits der Bezugslinie 22 liegende Rahmen gestaltet werden und im elastischen Verhalten insbesondere dem Kortikalknochen auf der lingualen Seite des Kiefers ähnlich gestaltet werden. Insofern kann der in Blickrichtung von Figur 7 auf der linken Seite von Linie 22 liegende Rahmenteil im Sinne der Erfindung als ein Baubereich 24 mit einem zugeordneten Materialverhalten und der in Blickrichtung rechts der Linie 22 liegende äußere Rahmen als ein weiterer Baubereich 25 mit von dem Baubereich 24 unterschiedlichen Materialeigenschaften betrachtet werden. Die Fußteilseite, die durch die Abrundung der lingualen Kieferseite angepasst ist, kann somit steifer als die gegenüberliegende, an die Wangenseite des Kieferknochens angepasste Fußteilseite im Bereich des Außenstegs 17 sein. Mit Bezug auf Figur 5 wurde ausgeführt, dass der Schaft 3 als senkrechter Gewindeträger mittels der darin eingebrachten gewendelten Inklusionen 7 eine im Vergleich zu dem Fußteil 2 als basale Platte geringere Sprödigkeit und höhere Elastizität besitzen kann. Aus Figur 7 geht hervor, dass auch am Fußteil 2 im Bereich des brückenartigen Innensteges 19, der einen den Gewindeträger 3 tragenden Querzug bildet, gewindeartig gewendelte Filamente 7 in den umgebenden Kunststoff eingearbeitet sind, um den Querzug 19 vergleichsweise elastischer zu gestalten. Diese Bereiche können insofern im Sinne der Erfindung als Baubereiche 26 mit von anderen Baubereichen innerhalb des gleichen Bauteils unterschiedlichen Materialeigenschaften aufgefasst werden.

Die Figuren 8 und 9 zeigen bevorzugte Abwandlungen von dem in Figur 7 gezeigten Fußteil 2. In Figur 8 wurde der Innensteg 19 im Vergleich zu Figur 7 von dem gerundeten Längsende um etwa ein Drittel der Gesamtlänge in Richtung auf das im Wesentlichen gerade begrenzte Längsende versetzt, so dass der Abstand nun von dort etwa ein Drittel der Gesamtlänge beträgt.

Figur 9 betrifft eine Ausführung, bei der beide der in den Figuren 7 und 8 gezeigten Innenstege 19 vorhanden sind.

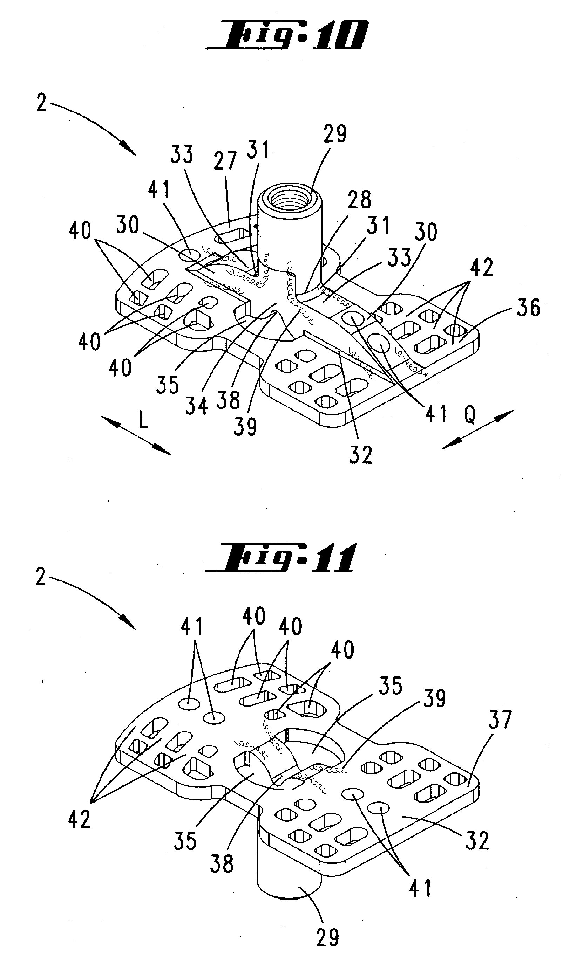

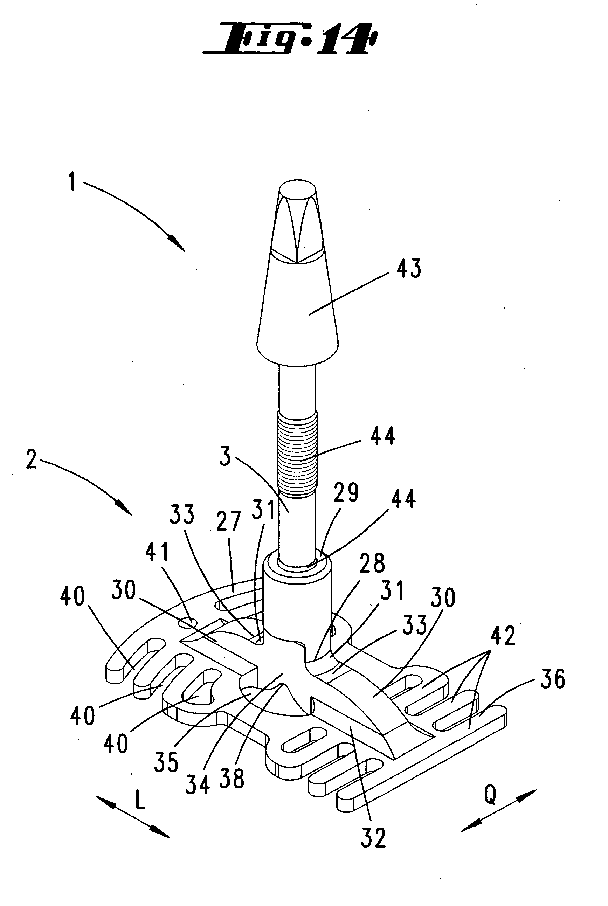

Die Figuren 10 und 11 zeigen perspektivisch ein Fußteil 2 gemäß einer weiteren bevorzugten Ausführungsform. Dieses ist einstückig ausgeführt und besitzt ein plattenartiges Basisteil 27 und ein Anschlussstück 28, speziell ein Gewindeanschlussstück 29 zum Anschließen eines nicht mit dargestellten Schafts (vgl. aber z. B. Figur 14), eines Abutments oder dergleichen. Des weiteren sind zwei über das plattenartige Basisteil erhabene Verstärkungsstege 30 vorhanden, die jeweils an ihrem zur Fußteilmitte gewandten Längsende 31 in das Anschlussstück 28 übergehen und die sich in Richtung eines in Längsrichtung L des Basisteils 27 orientierten Mittenbereichs 32 erstrecken. Der obere Rand 33 der Verstärkungsstege 30 fällt ab einem gewissen Abstand mit weiter zunehmendem Abstand von dem Anschlussstück 28 bis zur in Blickrichtung von Figur 10 oberen Oberfläche 36 des Basisteils gerundet ab. Die Länge der Verstärkungsstege 30 ist unterschiedlich, wobei der einem konvex gerundeten, in Einschubrichtung des Implantats vorderen Querrand zugewandte Verstärkungssteg kürzer ist und einen stegfreien Rand des Basisteils 27 lässt. Die Verstärkungsstege 30 gehen in einen zentralen Verbindungsbereich 34 ineinander über. Auf dessen beiden Seiten weist das Basisteil 27 etwa halbkreisförmig konturierte Mittendurchgangsöffnungen 35 auf. Die auf der den Verstärkungsstegen 30 gegenüberliegenden Seite des Fußteils liegende Oberfläche 37 des Basisteils besitzt in dem Verbindungsbereich 34 eine in Längsrichtung L symmetrisch bis zu einem schmalen Scheitel gerundete Eintiefung 38, die sich bis über die den Verstärkungsrippen zugewandte Plattenoberfläche 36 hinaus in den Verbindungsbereich 34 hinein erstreckt. Die Verstärkungsstege 30 besitzen dadurch insgesamt gleichsam die Form von Schenkeln, die zur gleichmäßigen Lastübertragung eines angeschlossenen Schafts oder dergleichen auf das Basisteil dienen und die dabei insbesondere auch die Funktion von federnden Trägern übernehmen können. In dem gewählten Ausführungsbeispiel sind dazu im Bereich der Verstärkungsstege zur Unterstützung dieser Eigenschaft Inklusionen aus schraubenartig gewendeltem Fasermaterial 39 in den umgebenden Werkstoff, bei dem es sich um Polyetheretherketon (PEEK) handelt, eingebettet. Wie ersichtlich, weisen die Verstärkungsstege einen rechteckigen Querschnitt auf. Schematisch ist angedeutet, dass sich das Fasermaterial 39 in seiner Längserstreckung überwiegend in Längsrichtung L der Verstärkungsstege erstreckt und an den Rändern bis in das Basisteil 27 in das Gewindeanschlussstück 29 reicht. Das plattenartige Basisteil 2 weist Durchgangsöffnungen 40 auf. In den vier Eckbereichen ist jeweils eine Gruppe aus sechs Öffnungen 40 gebildet, die jeweils paarweise in Längsrichtung L und innerhalb von Paaren in Querrichtung Q zueinander beabstandet sind. Durch die Durchgangsöffnungen werden Rippen 42 berandet, die im wesentlichen in Längsrichtung L verlaufen und die zur Anpassung an anatomische Gegebenheiten das Abtrennen (symmetrisch oder unsymmetrisch) von Bereichen des Basisteils erleichtern. Dies gilt entsprechend für die weiteren, dann quer, d. h. in Längsrichtung L verlaufenden Rippen. Entlang des Mittenbereichs 32 sind in dem Basisteil 27 vier zylindrische Durchgangsbohrungen 41 beabstandet vorgesehen, die sich je nach Position auch durch die Verstärkungsstege 30 erstrecken. Die Durchgangsbohrungen 41 können, wie noch nachfolgend erläutert, bei Bedarf wie auch das Gewindeanschlussstück 29 zum Anschluss von vorzugsweise stiftartigen Verbindungselementen dienen, so dass sich mehrere Fußteile 2 auf vielseitige unterschiedliche Weise miteinander verbinden lassen.

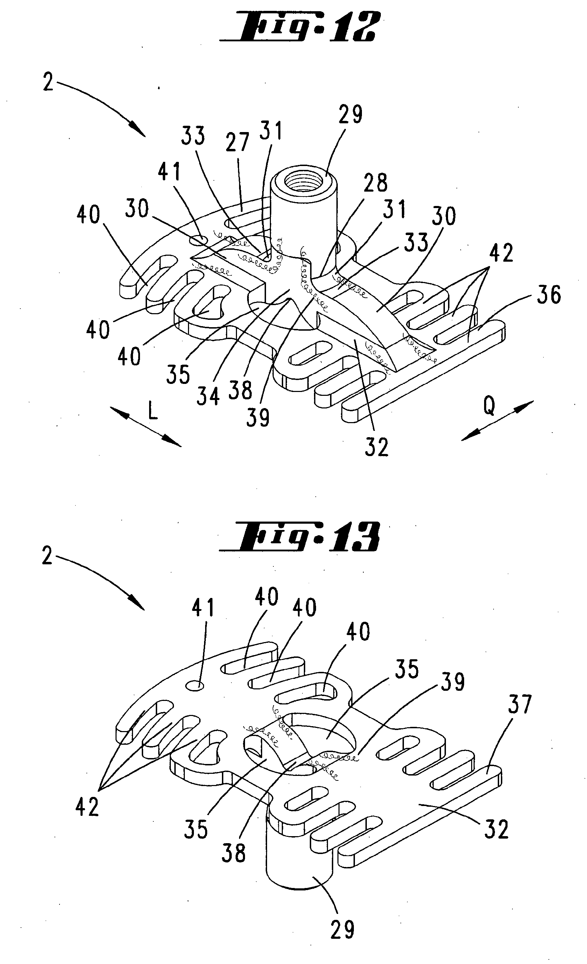

Die Figuren 12 und 13 zeigen eine im Rahmen der Erfindung weitere bevorzugte Ausführungsform des Fußteils 2, wobei hier wie im Vorangehenden für entsprechende bzw. vergleichbare Einzelheiten zur Übersicht die gleichen Bezugszeichen beibehalten werden. Der Unterschied zu der vorangehenden Ausführungsform liegt darin, dass die in Längsrichtung L äußeren Rippen 42 freie Enden aufweisen.

Figur 14 zeigt ein erfindungsgemäßes Implantat 1 gemäß einer weiteren bevorzugten Ausführungsform. Dabei ist in das in den Figuren 12,13 gezeigte Fußteil 2 ein Schaft 3 mittels eines daran endseitigen Gewindeabschnitts 44 eingeschraubt. An dem gegenüberliegenden Längsende befindet sich ein konusförmiges sog. Abutment 43, das zur Aufnahme bzw. als Untergrund für eine Krone, ein Prothetikteil oder dergleichen geeignet ist. Der Schaft 3 besitzt auf etwa halber Länge noch einen zweiten Gewindezonenabschnitt 44, an dem im Bedarfsfall ein weiteres, mit Durchgangsgewinde versehenes Plattenteil (Etagenteil) höheneinstellbar gehalten werden könnte. In dem gewählten Beispiel sind das Fußteil 2 und der Schaft 3 aus einem Material auf Basis von Polyetheretherketon (PEEK) hergestellt. Der für den Schaft 3 verwendete Werkstoff besitzt einen Elastizitätsmodul von ca. 4 GPa. Demgegenüber wurde das Fußteil 2 insgesamt aus mittels (zeichnerisch nicht dargestellten) Karbonfasern, insbesondere mittels glatten bzw. ungewendelten Fasern, verstärktem Polyetheretherketon hergestellt und besitzt einen Elastizitätsmodul von etwa 11 GPa.

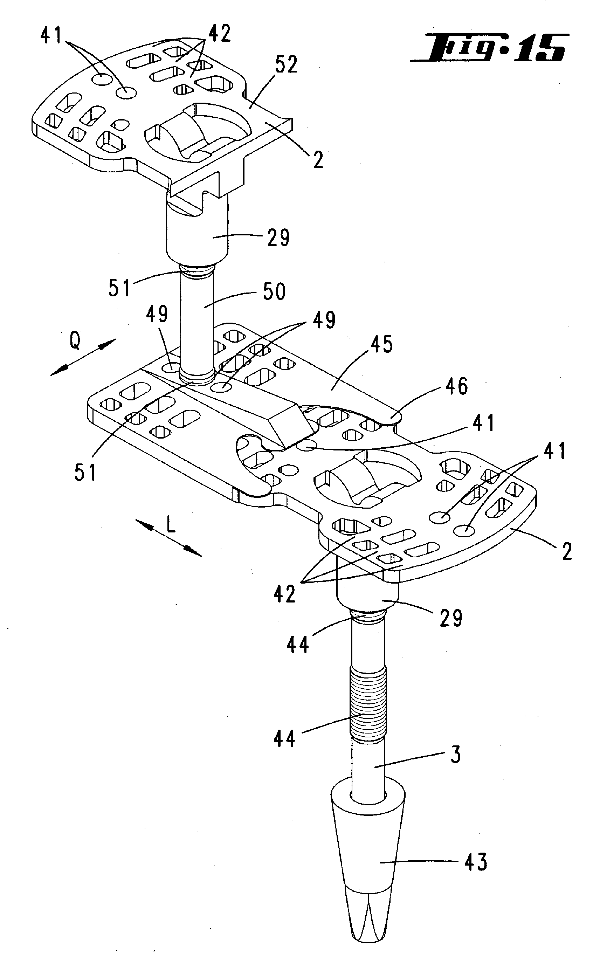

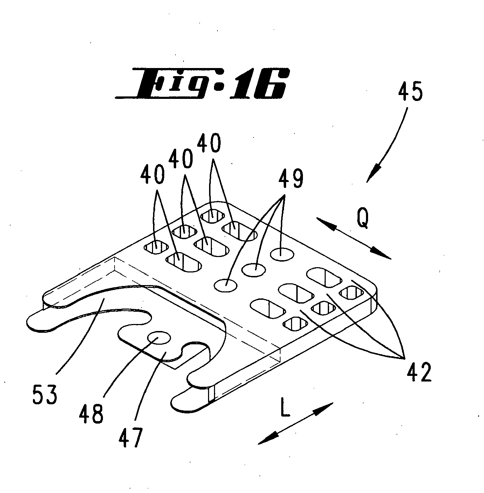

Figur 15 zeigt perspektivisch das erfindungsgemäße Implantat 1 gemäß einer weiteren bevorzugten Ausführungsform. Dieses weist auch den mit Bezug auf Figur 14 beschriebenen Schaft 3 auf, der in das zu den Figuren 10, 11 beschriebene Fußteil 2, speziell in dessen Gewindeanschlussstück 29, eingeschraubt ist. Dieses Fußteil 2 bildet gemeinsam mit dem auch in Figur 16 gezeigten Hülsenteil 45 eine erste, auch insgesamt scheibenartige Halterung 46. Das Hülsenteil 45 ist an dem Fußteil 2 formschlüssig gehalten, indem ein auf einer Federzunge 47 empor stehender kugelsegmentartiger Noppen 48 beim Einstecken des Fußteils zunächst federnachgiebig zurückweicht und dann bei Überdeckung durch die Federzunge in eine Durchgangsbohrung 41 eingedrückt wird. Zusätzlich ist das Hülsenteil 45 mit dem Fußteil 2 verklebt. Das Hülsenteil 45 weist in seinem Mittenbereich eine in Längsrichtung L orientierte Reihe aus drei beabstandeten Durchgangsbohrungen 49 auf. In dem gewählten Beispiel ist zur Anpassung an anatomische Gegebenheiten in die mittlere der drei Bohrungen 49 ein zylindrisches Verbindungselement 50 eingeschraubt, wobei mit dessen Gewindezonenabschnitt 51 selbstschneidend ein nicht dargestelltes Innengewinde in die Bohrung 49 eingeschnitten wurde. An dem anderen Längsende ist das Verbindungselement 50 in das Gewindeanschlussstück 29 eines weiteren Fußteils 2 eingeschraubt, welches eine zweite scheibenartige Halterung 52 bildet. Dessen Fußteil 2 unterscheidet sich von dem Fußteil 2 der ersten Halterung 46 dadurch, dass es zur Anpassung an anatomische Gegebenheiten in Längsrichtung gekürzt wurde. Die erste Halterung 46 kann vorzugsweise in eine in einem Kieferknochenbereich gefräste Nut zumindest plattenabschnittsweise lateral eingeschoben werden. Um zusätzlichen Halt zu erzeugen, kann die zweite Halterung 52 beispielsweise im Bereich des Jochbogens in eine weitere Fräsnut eingeschoben werden. Das Hülsenteil 45 dient zur Verlängerung der Verbindungsebene der ersten Halterung 46, d. h. ermöglicht einen größeren seitlichen Abstand zwischen den im Beispiel zueinander parallel orientierten Längsrichtungen des Schafts 3 und des Verbindungselements 50. Es versteht sich, dass je nach Anforderungen, wenn nur ein kleinerer Seitenabstand benötigt wird, auf das Hülsenteil 45 verzichtet werden könnte und das Verbindungselement 50 stattdessen beispielsweise in eine Durchgangsbohrung 41 des Fußteils 2 der ersten Halterung eingeschraubt werden könnte. Bei dem Gewindeanschlussstück 29 und bei den Durchgangsbohrungen 41 und 49 handelt es sich um insgesamt verteilt jeweils an den Halterungen 46, 52 liegende Anschlüsse, die wahlweise zum Anschließen des Schafts 3 und des Verbindungselements 50 geeignet sind. In dem gewählten Beispiel erstrecken sich der Schaft 3 und das Verbindungselement 50 jeweils senkrecht zu den Haupterstreckungsebenen der ersten und zweiten Halterung 46, 52 und auf zueinander parallel beabstandeten geometrischen Raumlinien.

Figur 16 verdeutlicht die an dem Hülsenteil 45 gebildete taschenartige Ausnehmung 53, deren hohler Taschenquerschnitt quer zur Längsrichtung L zur Erzielung einer formschlüssigen Steckhalterung an den Querschnitt des Fußteils 2 angepasst ist, d. h. etwa dessen Breite und Höhe aufweist. Bei dem in Figur 15 gezeigten Implantat sind der Schaft 3, das Verbindungsteil 50, die beiden Fußteile 2 und das Hülsenteil 45 aus einem Kunststoff, der Polyetheretherketon enthält, hergestellt. Der Schaft 3 und das Verbindungsteil 50 bestehen dabei aus homogenem Kunststoff und besitzen in dem gewählten Beispiel einen Elastizitätsmodul von etwa 4 GPa. Bei dem Hülsenteil 45 und dem Fußteil 2 der ersten Halterung 46 ist das Polyetheretherketon insgesamt glasfaserverstärkt, insbesondere unter Verwendung von glatten bzw. ungewendelten Fasern (zeichnerisch nicht dargestellt), und besitzt einen Elastizitätsmodul von etwa 11 GPa. Lediglich beispielhaft ist das Fußteil 2 der zweiten Halterung 52 karbonverstärkt (Fasern zeichnerisch nicht dargestellt) und besitzt ebenfalls einen Elastizitätsmodul von etwa 11 GPa. Alternativ könnte beispielsweise auch die erste Halterung 46 aus karbonverstärktem Polyetheretherketon bestehen, und es versteht sich, dass auch darüber hinaus zahlreiche weitere Abwandlungen möglich wären. Insbesondere können bei den Ausführungsbeispielen der Figuren 14,15 die Fußteile 2 zusätzlich die in den Figuren 10 -14 gezeigten gewendelten Fasern 39 aufweisen, oder auf diese kann verzichtet werden.

## Patentansprüche

1. Verfahren zur Bestimmung von lastabhängigen Verformungen in verschiedenen Knochenbereichen, bei welchem zum Vergleich Röntgenaufnahmen der Knochenbereiche ohne und mit mechanischer Belastung durchgeführt werden und bei welchem mittels Vergleich der mit und ohne Belastung aufgenommenen Röntgenaufnahmen für einzelne Knochenbereiche die dort unter der aufgebrachten Belastung stattgefundene Verformung ermittelt wird.

2. Verfahren nach Anspruch 1, **dadurch gekennzeichnet, dass** die Röntgenaufnahmen digitalisiert und rechnergestützt, insbesondere unter Verwendung von Bildverarbeitungssoftware, vergleichend ausgewertet werden.

3. Verfahren nach einem oder mehreren der vorangehenden Ansprüche, **dadurch gekennzeichnet, dass** anhand der Verformungen und der zugehörigen Belastungshöhe in verschiedenen Knochenbereichen jeweils der örtliche Wert einer Materialkenngröße für Verformungs- und/oder Festigkeitseigenschaften, insbesondere zur Charakterisierung des elastisches Knochenverhaltens, insbesondere der örtliche Wert des Elastizitätsmoduls, und/oder für das viskoelastische Knochenverhalten ermittelt wird.

4. Verfahren nach einem oder mehreren der vorangehenden Ansprüche, **dadurch gekennzeichnet, dass** Röntgen-Panorama-Aufnahmen von Kieferbereichen, insbesondere von ein Implantatbett umgebenden Knochenbereichen, mit und ohne Kaubelastung aufgenommen werden.

5. Verfahren nach einem oder mehreren der vorangehenden Ansprüche, **dadurch gekennzeichnet, dass** die örtlichen Werte zumindest einer Materialkenngröße für Verformungs- und/oder Festigkeitseigenschaften, insbesondere für den Elastizitätsmodul, für mehrere Randbereiche eines Implantatbettes in Knochen ermittelt werden und diese Werte dann zur Herstellung eines der Knochenumgebung des Implantatbetts angepassten Implantats (1), insbesondere basalen Kieferimplantats, verwendet werden, indem verschiedene, den ausgewerteten Randbereichen des Implantatbettes zugeordnete Baubereiche (5, 6, 12, 15, 24, 25, 26) des Implantats (1) hinsichtlich der Werte der Kenngröße den zugeordneten Randbereichen zumindest angenähert oder angepasst werden.

6. Implantat, insbesondere Kieferimplantat, welches mehrere, jeweils zur Implantation in oder zumindest benachbart zu Knochen geeignete Baubereiche aus nicht oder im Wesentlichen nicht von Knochen resorbierbarem Material aufweist, wobei zwei oder mehr dieser Baubereiche (5, 6, 12, 15, 24, 25, 26) zueinander unterschiedliche Materialeigenschaften, insbesondere zueinander unterschiedliche physikalische Eigenschaften, wie unterschiedliche Verformungs- und/oder Festigkeitseigenschaften, aufweisen, **dadurch gekennzeichnet, dass** das Implantat (1) mittels eines Verfahrens nach Anspruch 5 hergestellt ist,

7. Implantat nach Anspruch 6, **dadurch gekennzeichnet, dass** das Implantat (1) zumindest teilweise aus einem Baumaterial, welches Keton, insbesondere Polyetheretherketon oder Mischungen unterschiedlicher Polyetheretherketone, oder einen vergleichbaren Kunststoff wie Delrin enthält, hergestellt ist.

8. Implantat nach einem oder beiden der Ansprüche 6 und 7, **dadurch gekennzeichnet, dass** in einem oder mehreren Baubereichen (5, 6, 12, 5, 24, 25, 26) dem Kunststoff oder Keton, insbesondere dem Polyetheretherketon oder der Mischung aus Polyetheretherketonen, zumindest ein Zusatz, insbesondere faserartige Filamente (7, 19) insbesondere in Form von Kohlenstofffasern und/oder Glasfasern und/oder Titanfasern, und/oder ein oder mehrere nichtfaserige Zusätze beigegeben ist.

9. Implantat nach einem oder mehreren der Ansprüche 6 bis 8, **dadurch gekennzeichnet, dass** es sich bei dem Implantat (1) um ein ein- oder mehrteiliges, insbesondere um ein basal verankerbares, Kieferimplantat handelt.

## Claims

1. Method for determining load-dependent deformations in various bone regions, wherein X-ray images of the bone regions are taken without and with mechanical loading for comparison and wherein, by comparing the X-ray images for individual bone regions taken with and without loading, the deformation that has taken place there under the applied loading is determined.

2. Method according to claim 1, **characterised in that** the X-ray images are digitised and evaluated comparatively with the aid of computer technology, in particular using image processing software.

3. Method according to one or more of the preceding claims, **characterised in that** the location value of a characteristic material variable for deformation and/or strength properties, in particular for characterising the resilient bone behaviour, in particular the location value of the modulus of elasticity, and/or for the viscoelastic bone behaviour is determined in each case on the basis of the deformations and the associated level of loading in various bone regions.

4. Method according to one or more of the preceding claims, **characterised in that** panoramic X-ray images of jaw regions, in particular of bone regions surrounding an implant bed, are taken with and without loading from chewing.

5. Method according to one or more of the preceding claims, **characterised in that** the location values of at least one characteristic material variable for deformation and/or strength properties, in particular for the modulus of elasticity, are determined for a plurality of peripheral regions of an implant bed in bone and these values are then used to produce an implant (1), in particular a basal jaw implant, that is adapted to the bone surroundings of the implant bed, various structural regions (5, 6, 12, 15, 24, 25, 26) of the implant (1) that are associated with the evaluated peripheral regions of the implant bed being at least approximated or adapted to the associated peripheral regions with regard to the values of the characteristic variable.

6. Implant, in particular a jaw implant, which has a plurality of structural regions which are made of a material that cannot be or substantially cannot be resorbed by bones and are each suitable for implantation in or at least alongside bones, two or more of these structural regions (5, 6, 12, 15, 24, 25, 26) having material properties that are different from one another, in particular physical properties that are different from one another, such as different deformation and/or strength properties, **characterised in that** the implant (1) is produced by means of a method according to claim 5.

7. Implant according to claim 6, **characterised in that** the implant (1) is produced at least in part from a structural material which contains ketone, in particular polyether ether ketone or mixtures of different polyether ether ketones, or a comparable plastics material such as Delrin.

8. Implant according to one or both of claims 6 and 7, **characterised in that**, in one or more structural regions (5, 6, 12, 15, 24, 25, 26), at least one additive, in particular fibrous filaments (7, 13), in particular in the form of carbon fibres and/or glass fibres and/or titanium fibres, and/or one or more non-fibrous additives is added to the plastics material or ketone, in particular the polyether ether ketone or the mixture of polyether ether ketones.

9. Implant according to one or more of claims 6 to 8, **characterised in that** the implant (1) is a one-part or multi-part jaw implant, in particular a jaw implant that can be basally anchored.

## Revendications

1. Procédé de détermination de déformations en fonction de la contrainte dans différentes régions osseuses, selon lequel des radiographies des régions osseuses sont effectuées sans et avec contrainte mécanique à des fins de comparaison et selon lequel par comparaison des radiographies enregistrées avec et sans contrainte pour des régions osseuses individuelles, la déformation qui y est survenue sous la contrainte appliquée est déterminée.

2. Procédé selon la revendication 1, **caractérisé en ce que** les radiographies sont numérisées et évaluées comparativement assistées par ordinateur, en particulier en utilisant des logiciels de traitement d'image.

3. Procédé selon une ou plusieurs des revendications précédentes, **caractérisé en ce que** la valeur locale d'un paramètre caractéristique de matériau pour des propriétés de déformation et/ou de résistance, en particulier pour caractériser le comportement osseux élastique, en particulier la valeur locale du module d'élasticité, et/ou pour le comportement osseux viscoélastique est déterminée sur la base des déformations et des degrés de contrainte associés dans différentes régions osseuses.

4. Procédé selon une ou plusieurs des revendications précédentes, **caractérisé en ce que** des radiographies panoramiques de régions de mâchoire, en particulier de régions osseuses entourant un lit d'implant, sont prises avec ou sans contrainte de mastication.

5. Procédé selon une ou plusieurs des revendications précédentes, **caractérisé en ce que** les valeurs locales d'au moins un paramètre caractéristique de matériau pour des propriétés de déformation et/ou de résistance, en particulier pour le module d'élasticité, sont déterminées pour plusieurs régions de bord d'un lit d'implant dans des os et ces valeurs sont alors utilisées pour fabriquer un implant (1), en particulier d'un implant de mâchoire basal, adapté à l'environnement osseux du lit d'implant, en ce sens que différentes régions structurales (5, 6, 12, 15, 24, 25, 26) de l'implant (1) associées aux régions de bord évaluées du lit d'implant sont au moins approximées ou adaptées aux régions de bord associées pour ce qui est des valeurs des paramètres caractéristiques.

6. Implant, en particulier implant de mâchoire, qui présente plusieurs régions structurales respectivement appropriées à l'implantation dans ou au moins à proximité d'os, composées de matériau non résorbable ou pratiquement non résorbable par des os, deux ou plus de deux de ces régions structurales (5, 6, 12, 15, 24, 25, 26) présentant des propriétés de matériau différentes les unes des autres, en particulier des propriétés physiques différentes les unes des autres, telles que des propriétés de déformation et/ou de résistance différentes, **caractérisé en ce que** l'implant (1) est fabriqué par un procédé selon la revendication 5.

7. Implant selon la revendication 6, **caractérisé en ce que** l'implant (1) est fabriqué au moins en partie à partir d'un matériau structural qui contient de la cétone, en particulier du polyétheréthercétone ou des mélanges de différents polyétheréthercétones, ou une matière plastique comparable telle que du Delrin.

8. Implant selon une ou les deux revendications 6 et 7, **caractérisé en ce que** dans une ou plusieurs régions structurales (5, 6, 12, 15, 24, 25, 26), au moins un produit d'addition, en particulier des filaments fibreux (7, 13), en particulier sous forme de fibres de carbone et/ou de fibres de verre et/ou de fibres de titane, et/ou un ou plusieurs produits d'addition non fibreux sont ajoutés à la matière plastique ou à la cétone, en particulier au polyétheréthercétone ou au mélange de polyétheréthercétones.

9. Implant selon une ou plusieurs des revendications 6 à 8, **caractérisé en ce qu'**il s'agit, ce qui concerne l'implant (1), d'un implant de mâchoire en une ou plusieurs parties, en particulier d'un implant de mâchoire basal ancrable,
